# EUROPEAN PATENT APPLICATION

(11) **EP 4 549 907 A1**
(43) Date of publication of application: **07.05.2025**
(21) Application number: 22949157.6
(22) Date of filing: 19.12.2022
(51) Int. Cl.: G01N 1/28, G01N 1/02, H01L 21/67

(54) **METAL CONTAMINATION COLLECTING SYSTEM AND METAL CONTAMINATION COLLECTING METHOD**

(30) Priority: 29.06.2022 CN 202210750298
(71) Applicant: Jiangsu Leuven Instruments Co., Ltd., Xuzhou, Jiangsu 221300 (CN)
(72) Inventor: ZOU, Zhiwen, Xuzhou, Jiangsu 221300 (CN); ZHANG, Junhao, Xuzhou, Jiangsu 221300 (CN); HENG, Keji, Xuzhou, Jiangsu 221300 (CN); CHENG, Shiran, Xuzhou, Jiangsu 221300 (CN); MENG, Qingguo, Xuzhou, Jiangsu 221300 (CN); CUI, Hushan, Xuzhou, Jiangsu 221300 (CN); XU, Kaidong, Xuzhou, Jiangsu 221300 (CN)
(74) Representative: Eisenführ Speiser
(86) International application number: PCT/CN2022/139929
(87) International publication number: WO 2024/001081

(57) **Abstract**

A metal contamination collecting system and collecting method. The collecting system comprises a vapor phase corrosion chamber (10), and a first channel, a second channel, a third channel, and a fourth channel which are communicated with the vapor phase corrosion chamber (10), the first channel, the second channel, the third channel, and the fourth channel being independent of each other, and being respectively used for introducing, into the vapor phase corrosion chamber (10), a strong acid gas, an oxidizing gas, an inert gas, and a surface modification gas that enables the surface of a hydrophilic wafer to be hydrophobized. In the collecting process, the surface modification gas is introduced into the vapor phase corrosion chamber (10) so that the surface of the hydrophilic wafer is hydrophobized, thereby during metal contamination collecting, preventing a scanning liquid from dispersing and adhering to the surface of the wafer, and ensuring that the collecting system efficiently collects metal contamination.

## Description

This application claims priority to Chinese Patent Application No. 2022107502982, titled "METAL CONTAMINATION COLLECTING SYSTEM AND METAL CONTAMINATION COLLECTING METHOD", filed on June 29, 2022 with the China National Intellectual Property Administration, which is incorporated herein by reference in its entirety.

### FIELD

The present application relates to the technical field of semiconductors, and in particular to a metal contamination collecting system and a metal contamination collecting method.

### BACKGROUND

Driven by the strong momentum of Moore's Law, high-performance, low-power chips based on fin field-effect transistors (FinFET) of the 7nm technology generation have been integrated into today's smartphones. To manufacture such powerful chips, a large quantity of new chemical elements needs to be introduced. Each element has a different effect on silicon-based devices, some are necessary and are added to achieve functionality, while others are inadvertently introduced and become contaminant elements that reduce chip performance or even cause chip failure. Most of the contaminant elements are metal elements, which are referred to as metal contamination.

To ensure the chip performance, in the chip manufacturing process, a collection system is used to collect the metal contamination from hydrophilic surfaces of p++/n++ layers, hydrophilic films such as SiO₂, SiNₓ, and SiON films, and hydrophilic substrates such as quartz, glass or silicon carbide (SiC) substrates, and then the metal contamination is analyzed through an inductively coupled plasma mass spectrometry (ICP-MS).

At present, the collection system generally uses a nozzle based on a nitrogen wall to control a liquid droplet to collect the metal contamination. This collection system often encounter problems of collection failure or a collection rate lower than 50%, which make it impossible to perform further metal contamination analysis.

Therefore, how to achieve efficient collection of the metal contamination is a technical problem for those skilled in the art to solve.

### SUMMARY

In order to solve the above technical problem, a metal contamination collecting system is provided according to the present application. The metal contamination collecting system includes a vapor phase corrosion chamber, and a first channel, a second channel, a third channel and a fourth channel which are in communication with the vapor phase corrosion chamber. The first channel, the second channel, the third channel and the fourth channel are independent of each other, and are respectively for introducing a strong acid gas, an oxidizing gas, an inert gas, and a surface modification gas for making a hydrophilic surface of a wafer hydrophobic into the vapor phase corrosion chamber.

In an embodiment of the metal contamination collecting system, the metal contamination collecting system further includes a scanning chamber and a nozzle for spraying a scanning liquid into the scanning chamber.

In an embodiment of the metal contamination collecting system, the metal contamination collecting system further includes a gas extraction device in communication with the vapor phase corrosion chamber.

In an embodiment of the metal contamination collecting system, the metal contamination collecting system further includes an exhaust gas collection device in communication with the gas extraction device.

In an embodiment of the metal contamination collecting system, the exhaust gas collection device is filled with an alkaline absorption liquid.

In an embodiment of the metal contamination collecting system, the exhaust gas collection device is provided with a pH value indicator.

In an embodiment of the metal contamination collecting system, the metal contamination collecting system further includes a cooling device, and a chamber wall of the vapor phase corrosion chamber is provided with a cooling channel in communication with the cooling device.

In an embodiment of the metal contamination collecting system, a cooling liquid is accommodated in the cooling device, and a temperature of the cooling liquid is between 5 °C and 100 °C.

In an embodiment of the metal contamination collecting system, a specialty gas leakage sensor is provided close to the vapor phase corrosion chamber.

In an embodiment of the metal contamination collecting system, a wafer lifting device is provided in the vapor phase corrosion chamber.

In addition, a metal contamination collecting method is further provided according to the present application. The method includes the following steps: introducing a surface modification gas into a vapor phase corrosion chamber to make a hydrophilic surface of a wafer hydrophobic; then introducing an inert gas into the vapor phase corrosion chamber to discharge surface modification gas residue in the vapor phase corrosion chamber; and then spraying a scanning liquid onto the hydrophilic surface of the wafer to collect metal contamination. The scanning liquid is selected according to an element being monitored.

In an embodiment of the metal contamination collecting method, in a case that the element being monitored in the metal contamination is a noble metal element, before the step of introducing the surface modification gas into the vapor phase corrosion chamber, the following steps are further performed: introducing a strong acid gas and an oxidizing gas into the vapor phase corrosion chamber to corrode the noble metal element from a metallic state to an ionic state, and then introducing the inert gas into the vapor phase corrosion chamber to discharge strong acid gas residue and oxidizing gas residue in the vapor phase corrosion chamber.

In an embodiment of the metal contamination collecting method, the surface modification gas is selected from octadecyltrichlorosilane, hexamethyldisilane, 1H,1H,2H,2H-perfluorooctyltrichlorosilane, and a mixture thereof.

In an embodiment of the metal contamination collecting method, the strong acid gas is selected from HCl, HNO₃, HBr, HI, HClO₃, and a mixture thereof.

In an embodiment of the metal contamination collecting method, the oxidizing gas is selected from O₃, H₂O₂, HClO₄, and a mixture thereof.

In an embodiment of the metal contamination collecting method, before a step of detecting and analyzing the element in the metal contamination being collected, the method includes a step of adding an alkaline solution to the scanning liquid containing the noble metal element.

In an embodiment of the metal contamination collecting method, the alkaline solution is selected from ammonia solution, tetramethylammonium hydroxide, corrin, and a mixture thereof.

In an embodiment of the metal contamination collecting method, in a case that the noble metal element being monitored is Ag, the scanning liquid is nitric acid solution, and a ratio of the nitric acid solution is: HNO₃:H₂O = 1:100.

In an embodiment of the metal contamination collecting method, in a case that the noble metal element being monitored is Au, the scanning liquid is hydrochloric acid solution or nitric acid solution, a ratio range of the hydrochloric acid solution is: HCl:H₂O = 1:1 to 10:1, and a ratio range of the nitric acid solution is: HCl:H₂O = 1:1 to 10:1.

In an embodiment of the metal contamination collecting method, in a case that the noble metal element being monitored is Pt, the scanning liquid is hydrochloric acid solution, and a ratio range of the hydrochloric acid solution is: HCl:H₂O = 1:1 to 1:50.

The metal contamination collection system according to the present application is provided with the channel for introducing the surface modification gas into the vapor phase corrosion chamber. During the collecting process, the surface modification gas is introduced into the vapor phase corrosion chamber to make the hydrophilic surface of the wafer hydrophobic. In this way, when collecting the metal contamination, the scanning liquid will not disperse and adhere to the wafer surface due to the hydrophilicity of the wafer surface. If the scanning liquid disperses and adheres to the wafer surface, it will cause collection failure or low collection rate. Therefore, introducing the surface modification gas into the vapor phase corrosion chamber to make the hydrophilic surface of the wafer hydrophobic can ensure that the metal contamination collecting system collects the metal contamination efficiently.

### BRIEF DESCRIPTION OF THE DRAWINGS

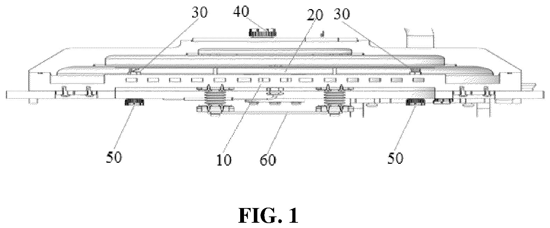

FIG.1 is a partial structural schematic view of a metal contamination collecting system according to an embodiment of the present application.

Reference numerals:

| | | | |
|---|---|---|---|
| 10: | vapor phase corrosion chamber, | 20: | scanning chamber, |
| 30: | nozzle, | 40: | gas extraction device connecting port, |
| 50: | cooling device connecting port, | 60: | wafer lifting device. |

### DETAILED DESCRIPTION OF EMBODIMENTS

In the past, a collection system generally used a nozzle based on a nitrogen wall to control a liquid droplet to collect metal contamination. This collection system often encounter problems of collection failure or a collection rate lower than 50%, which make it impossible to perform further metal contamination analysis.

To this end, a metal contamination collecting system and a metal contamination collecting method are provided according to the present application. The metal contamination can be efficiently collected to ensure a smooth progress of metal contamination analysis through use of the present application.

In order to enable those skilled in the art to better understand the technical solution of the present application, the metal contamination collecting system and the metal contamination collecting method according to the present application are further described in detail hereinafter in combination with accompanying drawings and specific embodiments.

Referring to FIG. 1, the metal contamination collecting system includes a vapor phase corrosion chamber 10, which may be made of corrosion-resistant polyvinylidene fluoride (PVDF) and sealed by a corrosion-resistant sealing ring.

The metal contamination collecting system is further provided with a first channel, a second channel, a third channel and a fourth channel, which are not shown in the figure. The first channel, the second channel, the third channel, and the fourth channel are all in communication with the vapor phase corrosion chamber 10. The first channel, the second channel, the third channel, and the fourth channel are independent of each other and are respectively for introducing a strong acid gas, an oxidizing gas, an inert gas (such as nitrogen), and a surface modification gas that makes a hydrophilic surface of a wafer hydrophobic into the vapor phase corrosion chamber 10. The first channel, the second channel, the third channel, and the fourth channel are independent of each other, so as to facilitate the individual control of the four gases.

Specifically, the strong acid gas is selected from HCl, HNO₃, HBr, HI, HClO₃, and a mixture thereof.

Specifically, the oxidizing gas is selected from O₃, H₂O₂, HClO₄, and a mixture thereof.

Specifically, the surface modification gas is selected from octadecyltrichlorosilane (ODTS), hexamethyldisilane (HMDS), 1H,1H,2H,2H-perfluorooctyltrichlorosilane (FOTS), and a mixture thereof.

As shown in FIG. 1, the metal contamination collecting system further includes a scanning chamber 20 and a nozzle 30 for spraying a scanning liquid into the scanning chamber 20, and metal contamination is collected by spraying the scanning liquid.

Further, as shown in FIG. 1, the metal contamination collecting system is further provided with a gas extraction device connecting port 40 to be connected to a gas extraction device. The gas extraction device is in communication with the vapor phase corrosion chamber 10 to assist in the discharge of the gas in the vapor phase corrosion chamber 10.

In addition, the metal contamination collecting system is further provided with an exhaust gas collection device in communication with the gas extraction device, and the exhaust gas collection device is filled with an alkaline absorption liquid (such as NaOH solution) to absorb an acidic gas discharged from the vapor phase corrosion chamber 10 to prevent the directly discharged acidic gas from being detrimental to the environment. The exhaust gas collection device may be provided with a pH value indicator to facilitate a user in monitoring a current pH value of a solution in the exhaust gas collection device. When the pH value is less than or equal to 5, the user is reminded to replace the solution in the exhaust gas collection device.

Further, as shown in FIG. 1, the metal contamination collecting system is further provided with a cooling device connecting port 50 to be connected to a cooling device. A cooling channel is provided in a chamber wall of the vapor phase corrosion chamber 10, and the cooling channel is in communication with the cooling device. The inside of the vapor phase corrosion chamber 10 is kept at a suitable temperature through introducing a cooling liquid into the cooling channel, which is conducive to improving a collection rate of the metal contamination. When collecting different metal contamination elements, the temperature requirements inside the vapor phase corrosion chamber 10 are different. Thus, a temperature of the cooling liquid may be determined according to specific process requirements, and the temperature of the cooling liquid generally ranges from 5 °C to 100 °C.

Further, as shown in FIG. 1, the metal contamination collecting system is further provided with a wafer lifting device 60 to facilitate the installation and removal of the wafer. In addition, a specialty gas leakage sensor is further provided close to the vapor phase corrosion chamber 10 to ensure the safe operation of the metal contamination collecting system.

During the collection process, a collection method varies depending on an element being monitored.

Specifically, for a common metal element, the collection method is as follows. The surface modification gas is firstly introduced into the vapor phase corrosion chamber 10 to make the hydrophilic surface of the wafer hydrophobic, then the inert gas is introduced into the vapor phase corrosion chamber 10 to discharge the surface modification gas remaining in the vapor phase corrosion chamber 10, and then the scanning liquid is sprayed into the scanning chamber 20 to collect the metal contamination.

The common metal element includes but is not limited to: lithium (Li), sodium (Na), magnesium (Mg), aluminum (Al), potassium (K), calcium (Ca), titanium (Ti), vanadium (V), chromium (Cr), manganese (Mn), iron (Fe), cobalt (Co), nickel (Ni), copper (Cu), zinc (Zn), molybdenum (Mo), barium (Ba), tungsten (W), and lead (Pb).

Specifically, for a noble metal element, the collection method is as follows. The strong acid gas and the oxidizing gas are firstly introduced into the vapor phase corrosion chamber 10 to corrode the noble metal elements from the metallic state into the ionic state, then the inert gas is introduced into the vapor phase corrosion chamber 10 to discharge the strong acid gas and the oxidizing gas remaining in the vapor phase corrosion chamber 10, then the surface modification gas is introduced into the vapor phase corrosion chamber 10 to make the hydrophilic surface of the wafer hydrophobic, then the inert gas is introduced into the vapor phase corrosion chamber 10 to discharge the surface modification gas remaining in the vapor phase corrosion chamber 10, and then the scanning liquid is sprayed into the scanning chamber 20 to collect the metal contamination.

The noble metal element includes but is not limited to: ruthenium (Ru), platinum (Pt), gold (Au), silver (Ag), and hafnium (Hf).

Whether it is a common metal element or a noble metal element, during the collection process, the surface modification gas is introduced into the vapor phase corrosion chamber 10 to make the hydrophilic surface of the wafer hydrophobic. In this way, when collecting the metal contamination, the scanning liquid will not disperse and adhere to the wafer surface due to the hydrophilicity of the wafer surface. If the scanning liquid disperses and adheres to the wafer surface, it will cause collection failure or low collection rate. Therefore, the surface modification gas is introduced into the vapor phase corrosion chamber 10 to make the hydrophilic surface of the wafer hydrophobic, which can ensure that the metal contamination collecting system collects the metal contamination efficiently.

Specifically, the scanning liquid is selected according to the element being monitored.

In a case that the element being monitored is Ag, the scanning liquid is nitric acid solution. Preferably, a ratio of the nitric acid solution is: HNO₃:H₂O = 1:100.

In a case that the element being monitored is Au, the scanning liquid is hydrochloric acid solution or nitric acid solution. Preferably, a ratio of the hydrochloric acid solution is HCl:H₂O = 1:1 to 10:1, and a ratio of the nitric acid solution is HCl:H₂O = 1:1 to 10:1.

In a case that the element being monitored is Pt, the scanning liquid is hydrochloric acid solution, and a ratio of the hydrochloric acid solution is HCl:H₂O = 1:1 to 1:50.

For Ag, Au and Pt elements, a high collection rate can be achieved with the above-mentioned scanning liquids that have the above-described ratios respectively.

After the collection, the scanning liquid containing the metal contamination may be detected and analyzed through an inductively coupled plasma mass spectrometry (ICP-MS).

Before performing detection and analysis on the scanning liquid containing the noble metal element, an alkaline solution is added into the scanning liquid containing the noble metal element. In the alkaline solution, the noble metal can form a ligand with ammonia to improve the accuracy of the detection and analysis. In addition, the pH value of the scanning liquid can be reduced to about 5 to 8, so as to prevent the scanning liquid from corroding a detection head of the mass spectrometry and affecting the service life of the mass spectrometry. Specifically, the alkaline solution may be selected from ammonia solution, tetramethylammonium hydroxide (TMAH), corrin (coline), and a mixture thereof.

The principle and implementation of the present application are described hereinabove through specific examples. The description of the above embodiments is merely to facilitate understanding of the method of the present application and the core idea thereof. It should be noted that for those skilled in the art, a few of improvements and modifications may be made to the present application without departing from the principle of the present application, and these improvements and modifications are also deemed to fall within the scope of protection defined by the claims of the present application.

## Claims

1. A metal contamination collecting system, comprising:
a vapor phase corrosion chamber (10); and
a first channel, a second channel, a third channel, and a fourth channel, which are in communication with the vapor phase corrosion chamber (10), wherein
the first channel, the second channel, the third channel, and the fourth channel are independent of each other and are respectively for introducing a strong acid gas, an oxidizing gas, an inert gas, and a surface modification gas for making a hydrophilic surface of a wafer hydrophobic into the vapor phase corrosion chamber (10).

2. The metal contamination collecting system according to claim 1, further comprising a scanning chamber (20) and a nozzle (30) for spraying a scanning liquid into the scanning chamber (20).

3. The metal contamination collecting system according to claim 1, further comprising a gas extraction device in communication with the vapor phase corrosion chamber (10).

4. The metal contamination collecting system according to claim 3, further comprising an exhaust gas collection device in communication with the gas extraction device.

5. The metal contamination collecting system according to claim 4, wherein the exhaust gas collection device is filled with an alkaline absorption liquid.

6. The metal contamination collecting system according to claim 4, wherein the exhaust gas collection device is provided with a pH value indicator.

7. The metal contamination collecting system according to claim 1, further comprising a cooling device, wherein a chamber wall of the vapor phase corrosion chamber (10) is provided with a cooling channel in communication with the cooling device.

8. The metal contamination collecting system according to claim 7, wherein a cooling liquid is accommodated in the cooling device, and a temperature of the cooling liquid is between 5 °C and 100 °C.

9. The metal contamination collecting system according to any one of claims 1 to 8, wherein a specialty gas leakage sensor is provided close to the vapor phase corrosion chamber (10).

10. The metal contamination collecting system according to any one of claims 1 to 8, wherein a wafer lifting device (60) is provided in the vapor phase corrosion chamber (10).

11. A metal contamination collecting method, comprising the following steps:
introducing a surface modification gas into a vapor phase corrosion chamber (10) to make a hydrophilic surface of a wafer hydrophobic;
introducing an inert gas into the vapor phase corrosion chamber (10) to discharge surface modification gas residue in the vapor phase corrosion chamber (10); and
then spraying a scanning liquid onto the hydrophilic surface of the wafer to collect metal contamination, wherein
the scanning liquid is selected according to an element being monitored.

12. The metal contamination collecting method according to claim 11, wherein the element being monitored in the metal contamination is a noble metal element, and before the step of introducing the surface modification gas into the vapor phase corrosion chamber (10), the following steps are performed:
introducing a strong acid gas and an oxidizing gas into the vapor phase corrosion chamber (10) to corrode the noble metal element from a metallic state to an ionic state; and
then introducing the inert gas into the vapor phase corrosion chamber (10) to discharge strong acid gas residue and oxidizing gas residue in the vapor phase corrosion chamber (10).

13. The metal contamination collecting method according to claim 12, wherein the surface modification gas is selected from octadecyltrichlorosilane, hexamethyldisilane, 1H,1H,2H,2H-perfluorooctyltrichlorosilane, and a mixture thereof.

14. The metal contamination collecting method according to claim 12, wherein the strong acid gas is selected from HCl, HNO₃, HBr, HI, HClO₃, and a mixture thereof.

15. The metal contamination collecting method according to claim 12, wherein the oxidizing gas is selected from O₃, H₂O₂, HClO₄, and a mixture thereof.

16. The metal contamination collecting method according to claim 12, comprising the following steps:
adding an alkaline solution to the scanning liquid containing the noble metal element; and
then detecting and analyzing the element in the metal contamination being collected.

17. The metal contamination collecting method according to claim 16, wherein the alkaline solution is selected from ammonia solution, tetramethylammonium hydroxide, corrin, and a mixture thereof.

18. The metal contamination collecting method according to claim 12, wherein the noble metal element being monitored is Ag, the scanning liquid is nitric acid solution, and a ratio of the nitric acid solution is: HNO₃:H₂O = 1:100.

19. The metal contamination collecting method according to claim 12, wherein the noble metal element being monitored is Au, the scanning liquid is hydrochloric acid solution or nitric acid solution, a ratio range of the hydrochloric acid solution is: HCl:H₂O = 1:1 to 10:1, and a ratio range of the nitric acid solution is: HCl:H₂O = 1:1 to 10:1.

20. The metal contamination collecting method according to claim 12, wherein the noble metal element being monitored is Pt, the scanning liquid is hydrochloric acid solution, and a ratio range of the hydrochloric acid solution is: HCl:H₂O = 1:1 to 1:50.
